# EUROPEAN PATENT SPECIFICATION

(11) **EP 4 376 908 B1**
(45) Date of publication and mention of the grant of the patent: **07.10.2026**
(21) Application number: 22848802.9
(22) Date of filing: 29.07.2022
(51) Int. Cl.: A61L 27/60, A61L 27/58, A61L 27/18

(54) **COMBINED MACRO AND MICRO-POROUS HYBRID-SCALE FIBER MATRIX**
KOMBINIERTE MAKRO- UND MIKROPORÖSE FASERMATRIX IN HYBRIDGRÖSSE
MATRICE DE FIBRES COMBINÉES MACRO ET MICROPOREUSES À L'ÉCHELLE HYBRIDE

(30) Priority: 29.07.2021 US 202163203731 P
(43) Date of publication of application: 05.06.2024
(73) Proprietor: Acera Surgical, Inc., St. Louis, Missouri 63131 (US)
(72) Inventor: MACEWAN, Matthew R., St. Louis, Missouri 63131 (US); JENG, Lily, St. Louis, Missouri 63131 (US); RAHIMI, Abdolrasol, St. Louis, Missouri 63131 (US); JASSAL, Manisha, St. Louis, Missouri 63131 (US); KOVACS, Tamas, St. Louis, Missouri 63131 (US)
(74) Representative: Müller-Boré & Partner Patentanwälte PartG mbB
(86) International application number: PCT/IB2022/057029
(87) International publication number: WO 2023/007444

(56) References cited:
- EP-A2- 0 314 109
- US-A1- 2013 030 548
- US-A1- 2019 111 175
- US-A1- 2021 128 792
- US-B2- 10 149 749
- US-B2- 10 632 228
- JUN-HYEOG JANG ET AL: "Electrospun materials as potential platforms for bone tissue engineering", ADVANCED DRUG DELIVERY REVIEWS, vol. 61, 1 January 2009 (2009-01-01), pages 1065 - 1083, XP055009075, DOI: 10.1016/j.addr.2009.07.008
- MACEWAN MATTHEW R, MACEWAN SARAH, KOVACS TAMAS R, BATTS JOEL: "What Makes the Optimal Wound Healing Material? A Review of Current Science and Introduction of a Synthetic Nanofabricated Wound Care Scaffold", CUREUS, CUREUS INC., US, 2 October 2017 (2017-10-02), US , XP093030711, ISSN: 2168-8184, DOI: 10.7759/cureus.1736

## Description

### BACKGROUND

### Field

Embodiments of the disclosure generally relate to sheets of a hybrid-scale fiber matrix hybrid-scale fiber matrix having macro and micro pores for improving biocompatibility.

### Description

Numerous pathological conditions and surgical procedures result in substantial defects in a variety of organs, tissues, and anatomical structures. In the majority of such cases, surgeons and physicians are required to repair such defects utilizing specialized types of surgical meshes, materials, and/or scaffolds. Unfortunately, the in vivo performance of known surgical materials is negatively impacted by a number of limiting factors. For instance, existing synthetic surgical meshes typically result in excessive fibrosis or scarification leading to poor tissue integration and increased risk of post-operative pain. Simultaneously, known biologic materials may induce strong immune reactions and aberrant tissue ingrowth which negatively impact patient outcomes. Additionally, existing synthetic surgical meshes can create scarification, post-operative pain, limited mobility, limited range of motion, adhesions, infections, erosion, poor biomechanical properties, and/or poor intraoperative handling.

Nanofabricated, nanofiber, or hybrid-scale fiber matrices are meshes or materials composed of reabsorbable polymer fibers tens to thousands of times smaller than individual human cells, which have recently been proposed as a unique substrate for implantable surgical meshes and materials. Generally, existing nanofiber materials tend to possess suboptimal mechanical performance compared to known surgical meshes. Existing nanofiber materials do not possess the tensile strength, tear resistance, and burst strength needed for numerous surgical applications or for basic intraoperative handling prior to in vivo placement. To combat this deficiency, known meshes are formed using higher fiber densities as a means of improving mechanical strength. Yet, utilization of such high-density meshes can decrease effective cellular and tissue ingrowth into the mesh, decrease mesh integration with native tissue, and reduce the biocompatibility of the polymeric implant. As a result, nanofiber or hybrid-scale fiber matrix materials with increased thickness and/or strength and favorable cellular and/or tissue integration and biocompatibility is needed as well as a method for producing nanofiber materials.

Repairs to tissues may be facilitated by one or more materials as described herein, including sheets of polymeric materials or processed tissue that acts like the native tissue in question. For example, skin wounds, including those caused by trauma or deliberately during a medical procedure, may be repaired by application of materials with favorable cellular and tissue integration. To facilitate repair of skin wounds, dressings and other coverings may be applied in both clinical and surgical settings, to promote healing and protect the wound from further harm. Typical wound dressings have a variety of purposes, including absorption of exudate, drainage of exudate, management of exudate, debriding of foreign material and dead cellular matter, stemming bleeding, protecting from infection, and the easing of pain, as well as generally promoting the healing process. As another example, neurosurgical repairs may be effected using one or more materials described herein. Representative wound matrices are shown in: Macewan et al. (October 02, 2017) Cureus 9(10).

In addition while cell microarrays may be useful in biomedical research and tissue engineering, at least some known techniques for producing such cell microarrays may be costly and time consuming, and may require the use of specialized, sophisticated instrumentation.

### SUMMARY

The invention relates to sheets of a hybrid-scale fiber matrix having macro and micro pores for improving biocompatibility, as defined by the claims.

In particular, in some embodiments, a three-dimensional electrospun hybrid-scale fiber matrix is described for use in repairing tissue for wound care, the three-dimensional hybrid-scale fiber matrix comprises: a flexible electrospun fiber network, the flexible electrospun fiber network comprising: a first set of electrospun fibers comprising a first bioresorbable polymer; and a second set of electrospun fibers comprising a second bioresorbable polymer, wherein the first bioresorbable polymer comprises a different composition from the second bioresorbable polymer; the flexible electrospun fiber network further comprising one or more macro-scale pores and one or more micro-scale pores, the one or more macro-scale pores comprising an opening of 1 mm ± 10 % to 20 mm ± 10 %, and the one or more micro-scale pores comprising an opening with areas of 10 µm² ± 10 % to less than 300 µm², wherein the three-dimensional electrospun hybrid-scale fiber matrix is sufficiently flexible to facilitate application of the three-dimensional electrospun nanofiber synthetic skin graft to uneven surfaces of the tissue, wherein the three-dimensional electrospun nanofiber synthetic skin graft is sufficiently flexible to enable movement of the three-dimensional electrospun hybrid-scale fiber matrix by the tissue, wherein the first set of electrospun fibers comprise an average diameter less than 10 micrometers, optionally wherein the first set of electrospun fibers comprise an average diameter not greater than 2,000 nanometers, and wherein the first set of electrospun fibers and the second set of electrospun fibers are configured to degrade after application to the tissue.

**In** some embodiments of the three-dimensional electrospun hybrid-scale fiber matrix the one or more macro-scale pores of the three-dimensional electrospun hybrid-scale fiber matrix are configured to allow flow through or management of an exudate. In some embodiments of the three-dimensional electrospun hybrid-scale fiber matrix , the one or more micro-scale pores of the three-dimensional electrospun hybrid-scale fiber matrix are configured to facilitate cell growth.

In some embodiments, the three-dimensional electrospun hybrid-scale fiber matrix further comprises at least one projection arising from the surface. In some embodiments, of the three-dimensional electrospun hybrid-scale fiber matrix , the first bioresorbable polymer comprises poly(lactic-co-glycolic acid), and the second bioresorbable polymer comprises polydioxanone. In some embodiments, of the three-dimensional electrospun hybrid-scale fiber matrix , the perforations are distributed equally throughout the matrix.

Also described is a method of manufacturing a biomedical patch device for tissue repair, the method comprising: depositing a first structure of fibers having electrospun nanofibers via electrospinning, the first structure of fibers configured to promote cell growth; and depositing a second structure of fibers having electrospun nanofibers via electrospinning, the second structure of fibers configured to promote cell growth, the first structure of fibers comprising a different composition from the second structure of fibers; the first structure of fibers and the second structure of fibers comprising one or more macro-scale pores and one or more micro-scale pores, the one or more macro-scale pores comprising an opening of 1 mm ± 10 % 20 mm ± 10 %, and the one or more micro-scale pores comprising an opening with areas of 10 µm² ± 10 % to less than 300 µm²; the biomedical patch device comprising a surface,
wherein the surface comprises a surface pattern configured to contact tissue, wherein the surface pattern, the first structure of fibers, and the second structure of fibers are configured to promote cell growth in one or more defined directions, the biomedical patch device sufficiently flexible to facilitate application of the biomedical patch device to uneven surfaces of the tissue, the biomedical patch device sufficiently flexible to enable movement of the biomedical patch device with the tissue, wherein the first set of electrospun fibers comprise an average diameter less than 10 micrometers, optionally wherein the first set of electrospun fibers comprise an average diameter not greater than 2,000 nanometers, and wherein the first structure of fibers and the second structure of fibers are configured to degrade after application to the tissue.

In some embodiments of the method, a first portion of the biomedical patch of a particular size comprises a higher number of fibers than a second portion of the biomedical patch of the particular size. In some embodiments of the method, the surface pattern is formed by positioning a mask between a collector and a spinneret, wherein the mask is configured to prevent depositing at least some of the first structure of fibers or the second structure of fibers on the collector. In some embodiments of the method, the surface pattern is formed by depositing the first structure of fibers and the second structure of fibers directly on a collector without a mask. In some embodiments of the method, the surface pattern comprises a plurality of organized features. In some embodiments, the surface pattern comprises a plurality of topographical features configured to further promote migration of cells in one or more of the plurality of defined directions. In some embodiments, the macro-scale pores are generated through cutting, including mechanically, electronically, and/or computer controlled cutting. In some embodiments, the cutting is laser cutting. In some embodiments, the surface pattern further comprises projections arising from the surface. In some embodiments, the surface pattern further comprises indentations projecting below from the surface.

For purposes of this summary, certain aspects, advantages, and novel features of the invention are described herein. It is to be understood that not necessarily all such advantages may be achieved in accordance with any particular embodiment of the invention. Thus, for example, those skilled in the art will recognize that the invention may be embodied or carried out in a manner that achieves one advantage or group of advantages as taught herein without necessarily achieving other advantages as may be taught or suggested herein.

All of these embodiments are intended to be within the scope of the invention herein disclosed. These and other embodiments will become readily apparent to those skilled in the art from the following detailed description having reference to the attached FIGS.

### BRIEF DESCRIPTION OF THE DRAWINGS

**FIG.** 1 illustrates a scanning electron micrograph of an embodiment of a non-woven hybrid-scale fiber matrix of the disclosure.
**FIG.** 2 illustrates an embodiment of a macro and microporous sheet of hybrid-scale fiber matrix .
**FIGS. 3-10** illustrate different embodiments of macroporous patterns.
FIGS. **11A-11B** illustrate example perforations for embodiments of the disclosure.
**FIGS. 12A-12D** illustrate an embodiment of a macro and micro-porous sheet of hybrid-scale fiber matrix .
**FIGS. 13A-13E** illustrate example perforations for embodiments of the disclosure.
**FIGS. 14A-14B** illustrates example skin wounds. 14A illustrates a skin wound created by a laceration, while 14B illustrates a skin wound with avulsion and a meshed hybrid-scale fiber matrix covering.
**FIG. 15** illustrates an example skin wound and subsequent healing process over time.
**FIG. 16** illustrates an example skin wound and progression of healing over time.

### DETAILED DESCRIPTION

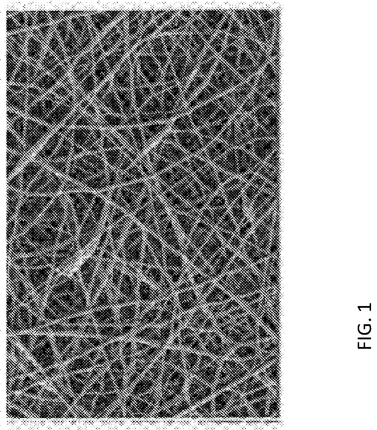

Embodiments provided herein facilitate repairing biological tissue or reinforcing biomedical material based on a biomedical patch (e.g., graft, nanofiber matrix, hybrid-scale fiber matrix , sheet) including a plurality of fibers, such as shown in FIG. 1. Such fibers may have a very small cross-sectional diameter (e.g., from 1-3000 nanometers, from 1-1000 nanometers) and, accordingly, may be referred to as nanofibers and/or microfibers. While biomedical patches are described herein with reference to dura mater and use as a surgical mesh, embodiments described may be applied to any biological tissue. In certain embodiments, the biomedical patches may be used as treatment for skin wounds. Moreover, although described as biomedical patches, structures with fibers may be used for other purposes. Accordingly, embodiments described are not limited to biomedical patches.

Patches may be described in further detail in U.S. Pat. Pub. Nos. 2017/0326270 and 2017/0319323, as well as U.S. Pat. No. 10,124,089, the entirety of each of which is hereby incorporated by reference in their entirety.

The hybrid-scale fiber matrix sheaths are both micro and macro-porous (e.g., meshed). The hybrid-scale fiber matrix itself can be microporous, and additional macro cuts/slits/pores/holes can be added into the matrix to provide an avenue allowing for exudate (e.g., liquids) to pass through the sheet. This can be particularly advantageous for wet wounds, as it can be problematic to have liquids trapped between the sheet and the wound. Excessive exudate buildup in a wound may lead to tissue damage and infection - therefore, adequate drainage and pass through of exudate may improve healing outcomes during wound treatment.

Advantageously, embodiments of the disclosed sheets can include a critical and specific cut pattern to be optimized for both cellular and tissue ingrowth in the material as well as fluid flow. Other physical properties can be optimized as well, such as stretching of the sheet to cover additional surface area strength, flexibility, and overall conformability, in addition to other properties. Further, embodiments of the disclosure can allow for the pattern to be controllable, and thus a user can modify the sheets as desired to optimize different properties.

Generally, the present disclosure is directed to non-woven graft materials including two or more distinct types of fiber compositions, each of which possesses independent mechanical, chemical and/or biological properties. Further, the material is both micro and macroporous. For example, in one embodiment, inclusion of one fiber composition can stabilize the resulting non-woven graft material, while the other fiber composition can improve stability, free-shrinkage properties, mechanical properties, and resorption rate of the non-woven graft material.

As used interchangeably herein, "non-woven graft material" and "non-woven graft fabric" refer to a material having a structure of individual fibers or threads which are interlaid, but not in an identifiable manner as in a knitted or a woven fabric. Non-woven graft materials and non-woven graft fabrics can be formed from many processes such as for example, electrospinning processes, meltblowing processes, spunbonding processes, melt-spraying and bonded carded web processes. The basis weight of non-woven graft materials is usually expressed in ounces of material per square yard (osy) or grams per square meter (gsm) and the fiber diameters are usually expressed in nanometers and micrometers (microns). Suitable basis weight of non-woven graft materials of the present disclosure can range from about 50 gsm to about 300 gsm. More suitably, basis weight of non-woven graft materials of the present disclosure can range from about 70 gsm to about 140 gsm. The tensile strength of the non-woven graft material of the present disclosure can range from about 5 Newtons (N) to about 50 Newtons (N), including from about 1 N to about 10 N to about 15 N. The strength of the non-woven graft material of the present disclosure can also be described in terms of suture pull-out strength, which refers to the force at which a suture can be torn from the non-woven graft material. Suitable suture pull-out strength can range from about 1 N to about 5 N.

As used herein the term "microfibers" refers to small diameter fibers having an average diameter not greater than 75 microns, for example, having an average diameter of from about 0.5 microns to about 50 microns, or more particularly, microfibers having an average diameter of from about 2 microns to about 40 microns. Another frequently used expression of fiber diameter is denier. The diameter of a polypropylene fiber given in microns, for example, may be converted to denier by squaring, and multiplying the result by 0.00629, thus, a 15 micron polypropylene fiber has a denier of about 1.42 (152×0.00629=1.415).

As used herein, the terms "nano-sized fibers" or "nanofibers" refer to very small diameter fibers having an average diameter not greater than 2000 nanometers, , not greater than 1500 nanometers, and suitably, not greater than 1000 nanometers (nm). Nanofibers are generally understood to have a fiber diameter range of about 10 to about 1500 nm, more specifically from about 10 to about 1000 nm, more specifically still from about 20 to about 500 nm, and most specifically from about 20 to about 400 nm. Other exemplary ranges include from about 50 to about 500 nm, from about 100 to 500 nm, or about 40 to about 200 nm.

As used herein the term "spunbonded fibers" refers to small diameter fibers which are formed by extruding molten thermoplastic material as filaments from a plurality of fine, usually circular capillaries of a spinneret with the diameter of the extruded filaments then being rapidly reduced as by, for example, in U.S. Pat. No. 4,340,563 to Appel et al., and U.S. Pat. No. 3,692,618 to Dorschner et al., U.S. Pat. No. 3,802,817 to Matsuki et al., U.S. Pat. Nos. 3,338,992 and 3,341,394 to Kinney, U.S. Pat. Nos. 3,502,763 and 3,909,009 to Levy, and U.S. Pat. No. 3,542,615 to Dobo et al.

As used herein the term "meltblown fibers" refers to fibers formed by extruding a molten thermoplastic material through a plurality of fine, usually circular, die capillaries as molten threads or filaments into converging high velocity gas (e.g. air) streams which attenuate the filaments of molten thermoplastic material to reduce their diameter, which may be to microfiber diameter. Thereafter, the meltblown fibers are carried by the high velocity gas stream and are deposited on a collecting surface to form randomly disbursed meltblown fibers. Such a process is disclosed, for example, in U.S. Pat. No. 3,849,241. Meltblown fibers are microfibers which may be continuous or discontinuous and are generally smaller than 10 microns in diameter.

As used herein, the term "electrospinning" refers to a technology which produces nano-sized fibers referred to as electrospun fibers from a solution using interactions between fluid dynamics and charged surfaces. In general, formation of the electrospun fiber involves providing a solution to an orifice in a body in electric communication with a voltage source, wherein electric forces assist in forming fine fibers that are deposited on a surface that may be grounded or otherwise at a lower voltage than the body. In electrospinning, a polymer solution or melt provided from one or more needles, slots or other orifices is charged to a high voltage relative to a collection grid. Electrical forces overcome surface tension and cause a fine jet of the polymer solution or melt to move towards the grounded or oppositely charged collection grid. The jet can splay into even finer fiber streams before reaching the target and is collected as interconnected small fibers. Specifically, as the solvent is evaporating (in processes using a solvent), this liquid jet is stretched to many times it original length to produce continuous, ultrathin fibers of the polymer. The dried or solidified fibers can have diameters of about 40 nm, or from about 10 to about 100 nm, although 100 to 500 nm fibers are commonly observed. Various forms of electrospun nanofibers include branched nanofibers, tubes, ribbons and split nanofibers, nanofiber yarns, surface-coated nanofibers (e.g., with carbon, metals, etc.), nanofibers produced in a vacuum, and so forth. The production of electrospun fibers is illustrated in many publication and patents, including, for example, P. W. Gibson et al., "Electrospun Fiber Mats: Transport Properties," AIChE Journal, 45(1): 190-195 (January 1999).

As used herein, the term "type" such as when referring to "different types of fibers" or "distinct types of fibers" refers to fibers having "a substantially different overall material composition" with measurably different properties, outside of "average diameter" or other "size" differences. That is, two fibers can be of the same "type" as defined herein, yet have different "average diameters" or "average diameter ranges." Although fibers are of different "types" when they have a substantially different overall material composition, they can still have one or more components in common. For example, electrospun fibers made from a polymer blend with a first polymeric component present at a level of at least 10 wt % would be considered a different fiber type relative to electrospun fibers made from a polymer blend that was substantially free of the first polymeric component. Fibers of different "types" can also have a completely different content, each made of a different polymer for example, or one made from a polymer fiber and the other from a titania fiber, or a ceramic fiber and a titania fiber, and so on.

As used herein the term "polymer" generally includes but is not limited to, homopolymers, copolymers, such as for example, block, graft, random and alternating copolymers, terpolymers, etc. and blends and modifications thereof. Furthermore, unless otherwise specifically limited, the term "polymer" shall include all possible geometrical configuration of the material. These configurations include, but are not limited to isotactic and atactic symmetries.

### Hybrid-Scale Fiber Graft Material

The non-woven graft materials of the present disclosure typically include at least two distinct types of fiber compositions, including compositions comprising hybrid-scale fiber matrices, each of which possesses independent mechanical, chemical and/or biological properties. However, in some embodiments the fibers can be the same. The fiber compositions are suitably made of synthetic resorbable polymeric materials. As used herein, the term "resorbable polymeric material" refers to material formed from resorbable (also referred to as "bioabsorbable") polymers; that is the polymers possess the property to break down when the material is exposed to conditions that are typical of those present in a post-surgical site into degradation products that can be removed from the site within a period that roughly coincides with the period of post-surgical healing. Such degradation products can be absorbed into the body of the patient. The period of post-surgical healing is to be understood to be the period of time measured from the application of the non-woven graft material of the present disclosure to the time that the post-surgical site is substantially healed. This period can range from a period of several days to several months depending on the invasiveness of the surgical and the speed of healing of the particular individual. It is intended that the subject non-woven graft material can be prepared so that the time required for resorption of the non-woven graft material can be controlled to match the time necessary for healing or tissue reformation and regeneration. For example, in some non-woven graft materials of the present disclosure, the fiber compositions are selected to degrade within a period of about one week, while in other non-woven graft materials, the compositions are selected to degrade within a period of three years, or even longer if desired.

The fiber compositions used in the present disclosure can be produced from any resorbable material that meets the criteria of that material as those criteria are described above. The fiber compositions can be formed from resorbable polymers such as (but not limited to) polymers of lactic and glycolic acids, copolymers of lactic and glycolic acids, poly(ether-co-esters), poly(hydroxybutyrate), polycaprolactone, copolymers of lactic acid and ε-aminocapronic acid, lactide polymers, copolymers of poly(hydroxybutyrate) and 3-hydroxyvalerate, polyesters of succinic acid, poly(N-acetyl-D-glucosamine), polydioxanone, cross-linked hyaluronic acid, cross-linked collagen, and the like, and combinations thereof. Suitable synthetic polymers can be, for example, polycaprolactone (poly(ε-caprolactone), PCL), polydioxanone (PDO), poly (glycolic acid) (PGA), poly(L-lactic acid) (PLA), poly(lactide-co-glycolide) (PLGA), poly(L-lactide) (PLLA), poly(D,L-lactide) (P(DLLA)), poly(ethylene glycol) (PEG), montmorillonite (MMT), poly(L-lactide-co-ε-caprolactone) (P(LLA-CL)), poly(ε-caprolactone-co-ethyl ethylene phosphate) (P(CL-EEP)), poly[bis(p-methylphenoxy) phosphazene] (PNmPh), poly(3-hydroxybutyrate-co-3-hydroxyvalerate) (PHBV), poly(ester urethane) urea (PEUU), poly(p-dioxanone) (PPDO), polyurethane (PU), polyethylene terephthalate (PET), poly(ethylene-co-vinylacetate) (PEVA), poly(ethylene oxide) (PEO), poly(phosphazene), poly(ethylene-co-vinyl alcohol), polymer nanoclay nanocomposites, poly(ethylenimine), poly(ethyleneoxide), poly vinylpyrrolidone; polystyrene (PS) and combinations thereof. Particularly suitable polymers include poly(lactic-co-glycolic acid), polydioxanone, polycaprolactone, and combinations thereof.

The fibers for the fiber compositions may be of a variety of sizes as deemed suitable by one skilled in the art for the end purpose of the non-woven graft material. In some embodiments, the fibers for the fiber composition may be a hybrid-scale fiber matrix. Typically, the fibers of a hybrid-scale fiber matrix have a mean fiber diameter of less than 5 µm, including less than 2 µm, including less than 1.5 µm, and including less than 1.0 µm. For example, in some embodiments, the fibers can have a mean fiber diameter ranging from about 10 nm to about 5 µm, more specifically from about 10 nm to about 1.0 µm, more specifically still from about 20 nm to about 500 nm, and most specifically from about 20 nm to about 400 nm. Other exemplary ranges include from about 50 nm to about 500 nm, from about 100 nm to about 500 nm, and about 40 nm to about 200 nm.

Suitable ratios of the first fiber composition to the second fiber composition resulting in the non-woven graft material can range from about 10 to 1 to about 1 to 10.

In some embodiments, the non-woven graft material is made from a first non-woven fiber composition prepared from poly(lactic-co-glycolic acid) and a second non-woven fiber composition prepared from polydioxanone. The resultant non-woven graft material is a non-biologic tissue substitute designed to provide optimal strength, handling, and suturability, while reducing local inflammation to provide improved wound healing and tissue regeneration. In an exemplary embodiment the non-woven graft material can be synthesized by electrospinning a first fiber composition including a copolymer of glycolide and L-lactide and a second fiber composition including polydioxanone (100 mol %) to create an architecture that is reminiscent of native extracellular matrix. The glycolide mol % to L-lactide mol % can range from about 100 mol % glycolide to 0 mol % L-lactide to 0 mol % glycolide to about 100 mol % L-lactide. A particularly suitable non-woven graft material includes a first fiber composition including a copolymer of glycolide and L-lactide having a glycolide mol % to L-lactide mol % ratio of 90 mol % glycolide and 10 mol % L-lactide. This method of synthesis creates a material that is mechanically strong, while providing the look and feel of native tissue. The architecture of this non-biologic graft material furthermore supports cellular and tissue ingrowth and neoduralization with minimal inflammation.

The non-woven graft material typically can be prepared to be any of a variety of sizes, shapes and thicknesses. Wet and dry non-woven graft material can suitably be cut and trimmed to any desired size and shape. In particularly suitable embodiments, the non-woven graft material has a size ranging from about 0.55 in diameter to about 0.5 in x 1 in to about 2.5 cm×2.5 cm (1 in×1 in) to about 25.5 cm×50 cm (10 in×20 in), including for example, from about 2.5 cm×2.5 cm (1 in×1 in), from about 5.0 cm×5.0 cm (2 in×2 in), from about 7.5 cm×7.5 cm (3 in×3 in), and including about 12.5 cm×17.5 cm (5 in×7 in), and including about 10 cm×12.5 (4 in×5 in).

The non-woven graft materials typically have a thickness ranging from about 0.1 mm to about 5 mm, including from about 0.3 mm to about 0.8 mm, about 0.3 mm to about 0.7 mm, and about 0.3 mm to about 0.5 mm

The non-woven graft material is typically microporous, and has interconnecting pores having a pore size in the range of from about 10 µm² to about 10,000 µm². Particularly suitable embodiments have a pore size of less than 300 µm². It is believed that pores of this size range can accommodate penetration by cells and can support the growth and proliferation of cells, followed by vascularization and tissue development.

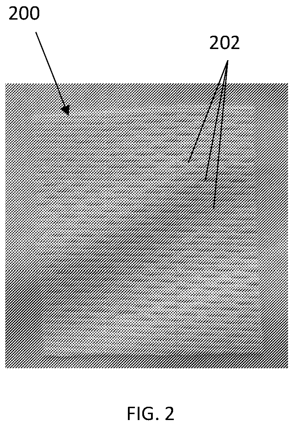

Additionally, as shown in FIG. 2, the material is macroporous as well as microporous. Thus, the non-woven hybrid-scale fiber matrix graft material sheet **200** can include one or more perforations (e.g., holes, cuts, slots, slits, apertures) **202** in the sheet **200.** In some embodiments, the perforations **202** can be found over the entire sheet **200.** In some embodiments, the perforations **202** may be formed only in a portion of the sheet **200,** such as 25%, 50%, or 75% of the sheet **200.** The sections with perforations **202** may be separated or connected. In some embodiments, the perforations **202** can be uniform, though in alternative embodiments they may not be. In some embodiments, the perforations **202** are generally equally distributed throughout the sheet **200,** which can provide equal distribution of fluid transport. In some embodiments, the perforations **202** can be concentrated in a certain area of the sheet **200,** thus making specific areas more capable of fluid transport than other areas. In some embodiments, the density of slits in the non-woven hybrid-scale fiber matrix graft material sheet may range from 1 to 400 slits per square inch. In some embodiments, the slits in the non-woven hybrid-scale fiber matrix graft material may comprise a density of about 1 slit per square inch, about 5 slits per square inch, about 10 slits per square inch, about 15 slits per square inch, about 20 slits per square inch, about 40 slits per square inch, about 60 slits per square inch, about 80 slits per square inch, about 100 slits per square inch, about 150 slits per square inch, about 200 slits per square inch, about 250 slits per square inch, about 300 slits per square inch, about 350 slits per square inch, about 400 slits per square inch, and/or may comprise a density within a range defined by two of the aforementioned values. In some embodiments, the length of perforations in the non-woven hybrid-scale fiber matrix graft material sheet may range from 1-20 mm. In some embodiments, the length of perforations may be about 1 mm, about 2 mm, about 3 mm, about 5 mm, about 10 mm, about 15 mm, about 20 mm, and/or may comprise slits with lengths within a range defined by two of the aforementioned values. In some embodiments, perforations may be separated into rows, wherein the distance between each row may range between 0.5-20 mm. In some embodiments, the distance between each row may be about 0.5 mm, about 1 mm, about 2 mm, about 3 mm, about 5 mm, about 10 mm, about 15 mm, about 20 mm, and/or may comprise a distance within a range defined by two of the aforementioned values. In some embodiments, the distance between individual perforations in a row may range between 1-20 mm. In some embodiments, the distance between individual perforations may be about 1 mm, about 2 mm, about 3 mm, about 5 mm, about 10 mm, about 15 mm, about 20 mm, and/or may comprise distances within a range defined by two of the aforementioned values.

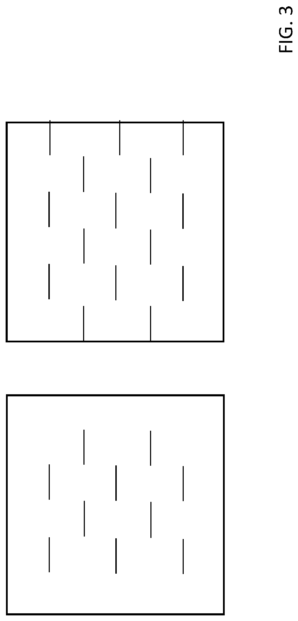

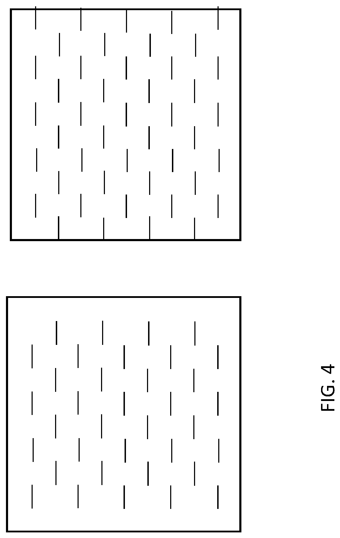

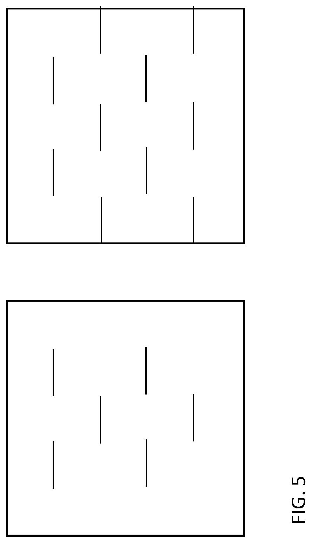

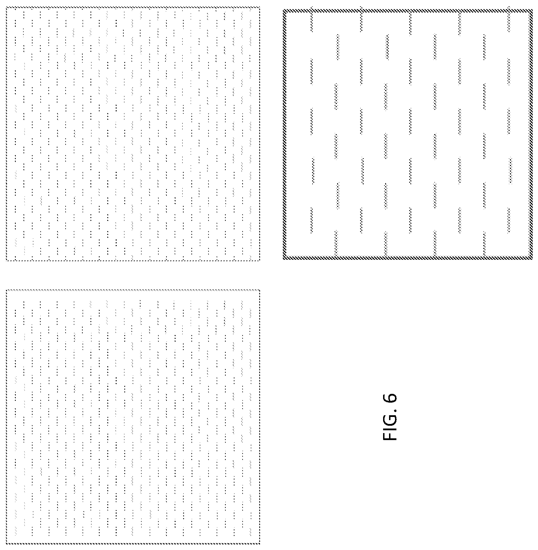

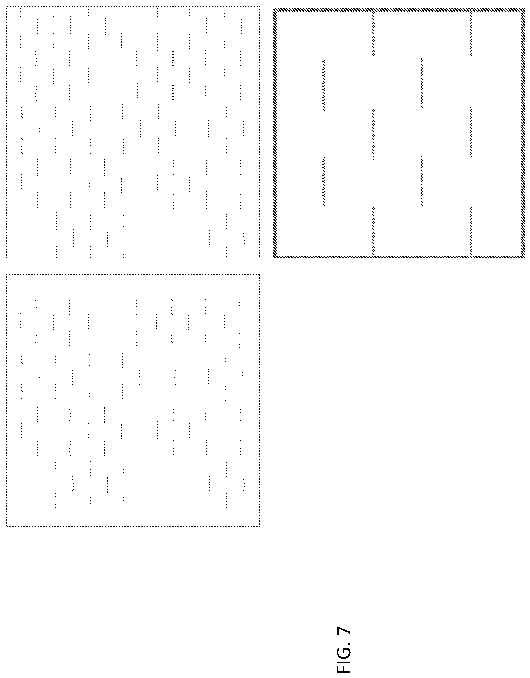

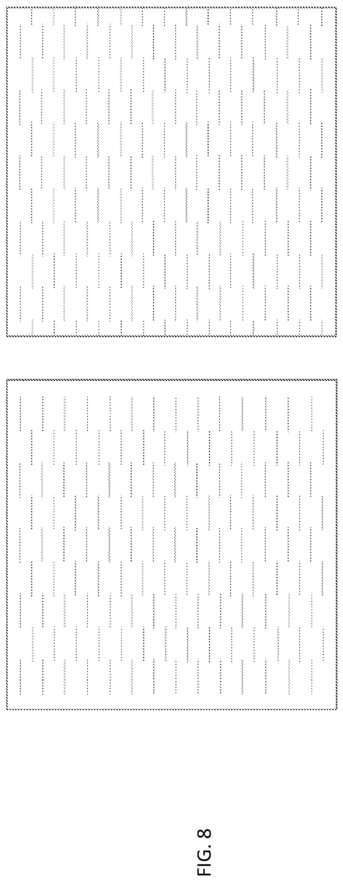

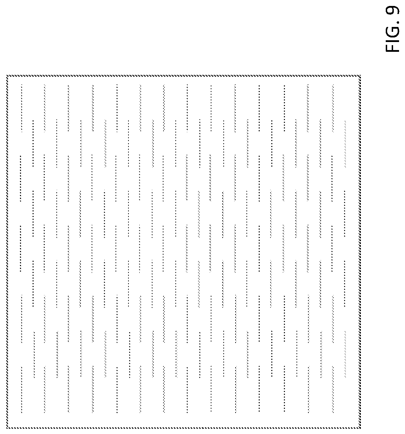

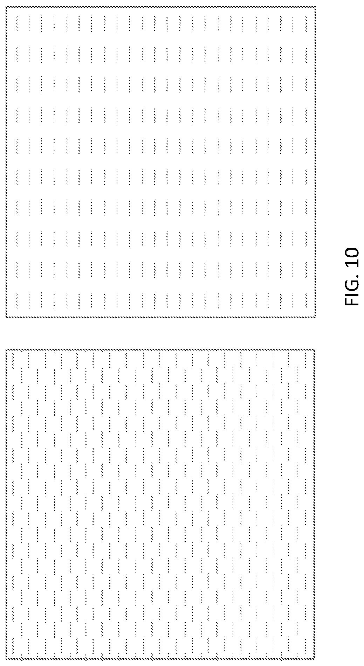

**FIGS. 3-10** illustrate non-limiting various perforation patterns that could be used with embodiments of the disclosure.

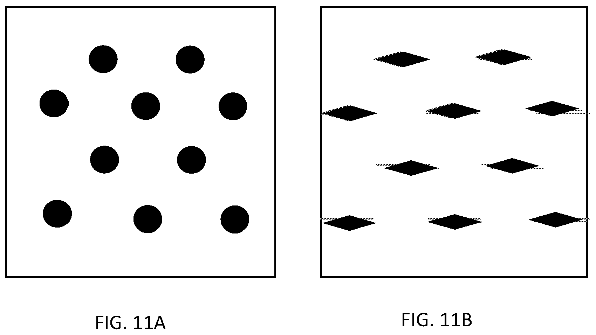

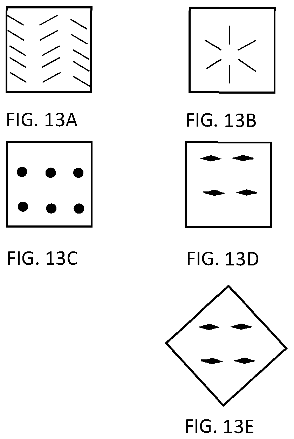

Further, while the previous FIGS. illustrate the perforations as straight line cuts, other types of perforations can be used as well. **FIGS. 11A-11B**-illustrate some examples of alternate perforations using circles **(****FIG. 11A****)** and diamonds **(****FIG. 11B****).** These are not limiting shapes, and other shapes can be used as well for the perforations, such as triangles, rectangles, ovals, irregular polymers, etc. Further, combinations of different types of perforations can also be used. **FIGS. 13A-13E** also disclose non-limiting example perforation patterns, including combinations of perforations to create chevrons and stars, as well as irregular shapes.

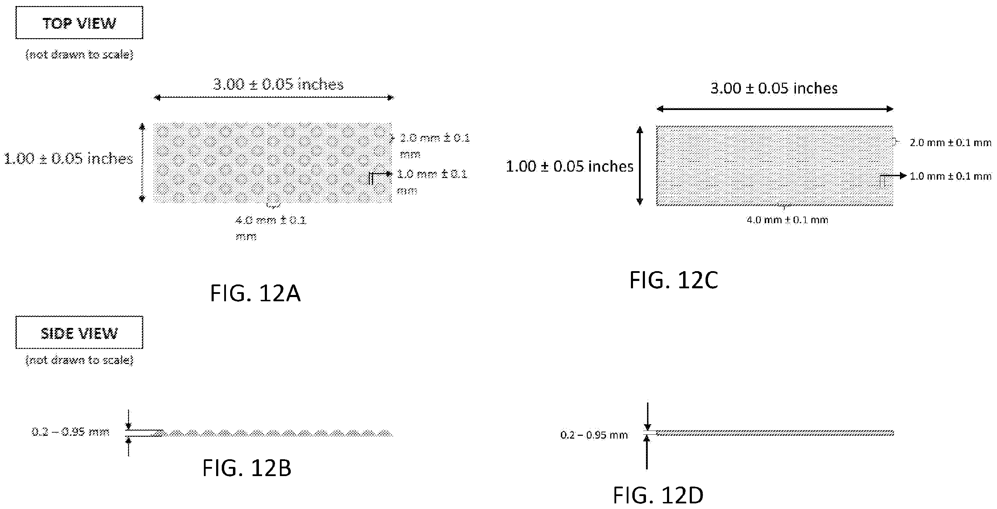

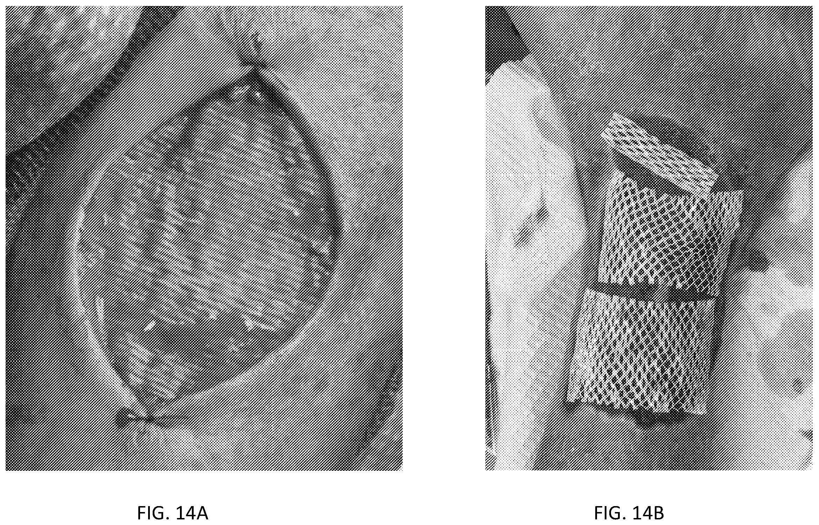

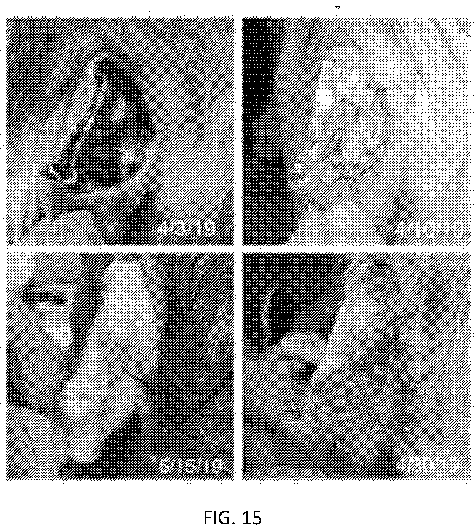

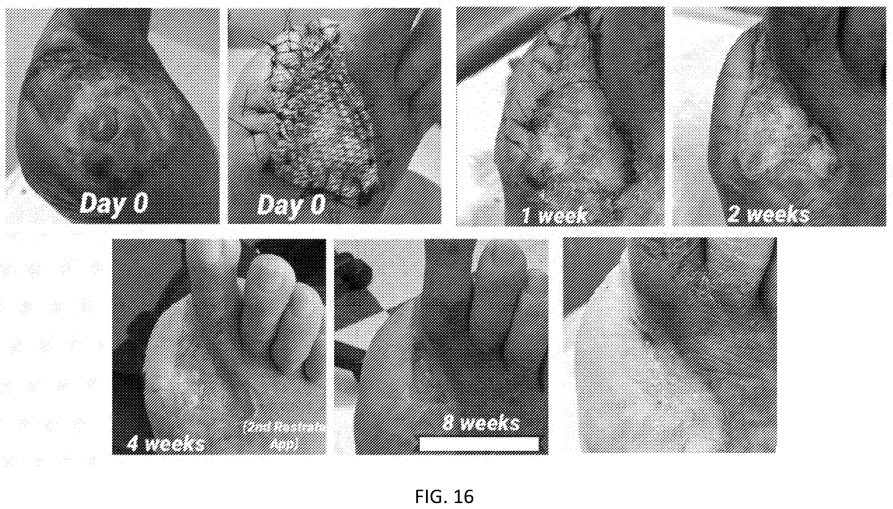

**FIGS. 12A-12D** illustrate embodiments of a macro and micro porous matrix as discussed herein. FIG. 12A illustrates a representational top view of an embodiment of the macro and micro porous matrix with dimensions of 3 ± .05 inches by 1 ± .05 inches. In FIG. 12A, the matrix is comprised of two fiber populations created by an electrospinning process wherein the first fiber population is a copolymer between glycolide and L-lactide, while the second fiber population is a polymer of paradioxanone. FIG. 12A further features a recurring pattern, featuring circular protrusions and perforations represented as white lines recurring over a substantial portion of the matrix. FIG. 12B is a depth representation side view of an embodiment of the macro and micro porous matrix, showing overall thickness of the matrix, ranging between 0.2 and 0.95 mm. FIG. 12. C illustrates a representational top view of an embodiment of the macro and micro porous matrix with dimensions of 3 ± .05 inches by 1 ± .05 inches. FIG. 12C features matrix is comprised of two fiber populations created by an electrospinning process wherein the first fiber population is a copolymer between glycolide and L-lactide, while the second fiber population is a polymer of paradioxanone. FIG. 12D is a depth representation side view of an embodiment of the macro and micro porous matrix, wherein the surface is substantially flat, or otherwise lacking in surface patterns. FIGS. 14A-14B, 15, and 16 illustrate non-limiting embodiments of the hybrid-scale fiber matrix as applied to tissue. FIGS. 14A and 14B illustrate a non-limiting embodiment wherein a non-woven graft material is overlaid onto a wound site.

**In** some aspects, the non-woven graft materials can be surface-modified with biomolecules such as (but not limited to) hyaluronans, collagen, laminin, fibronectin, growth factors, integrin peptides (Arg-Gly-Asp; i.e., RGD peptides), and the like, or by sodium hyaluronate and/or chitosan niacinamide ascorbate, which are believed to enhance cell migration and proliferation, or any combination thereof. The material can also be impregnated with these and other bioactive agents such as drugs, vitamins, growth factors, therapeutic peptides, and the like. In addition, drugs that would alleviate pain may also be incorporated into the material.

**In** another aspect, the present disclosure is directed to a laminate comprising a non-woven graft material, wherein the non-woven graft material includes a first non-woven fiber composition and a second non-woven fiber composition.

In some embodiments, the non-woven graft material of the laminate includes a first non-woven fiber composition including poly(lactic-co-glycolic acid) and a second non-woven fiber composition including polydioxanone, as described herein.

In some embodiments, the non-woven graft material can include at least one projection arising from a surface of the non-woven graft material. The projection is a protrusion or bulge arising from a surface of the non-woven graft material. The projection can arise from a top surface of the non-woven graft material, a bottom surface of the non-woven graft material, and a top surface and a bottom surface of the non-woven graft material. The projection can be any desired shape such as, for example, circular, spherical, square, rectangular, diamond, star, irregular, and combinations thereof. The projection can be any desired height as measured from the surface of the material to the top of the projection. In one embodiment, the projection can have a substantially uniform height from the surface of the material. In another embodiment, the projection can further form gradually from the surface of the material to the highest measurable surface of the projection. In some embodiments, a surface of the non-woven graft material includes a plurality of protrusions. The plurality of protrusions can be patterned or randomly distributed on a surface of the non-woven graft material. In another embodiment, the method includes forming at least one indentation in a surface of the non-woven graft material. The indentation is a recess or depression in a surface of the non-woven graft material. The indentation can in a top surface of the non-woven graft material, a bottom surface of the non-woven graft material, and a top surface and a bottom surface of the non-woven graft material. The indentation can be any desired shape such as, for example, circular, spherical, square, rectangular, diamond, star, irregular, and combinations thereof. The indentation can be any desired depth as measured from the surface of the material to the bottom of the indentation. In one embodiment, the indentation can have a substantially uniform depth from the surface of the material to the deepest depth of the indentation. In another embodiment, the indentation can further form gradually from the surface of the material to the deepest depth of the indentation. In some embodiments, a surface of the non-woven graft material includes a plurality of indentations. The plurality of indentations can be patterned or randomly distributed on a surface of the non-woven graft material. In another embodiment, the non-woven graft material can include at least one projection arising from a surface of the non-woven graft material and at least one indentation in the surface of the non-woven graft material. In another embodiment, the non-woven graft material can include at least one projection arising from a top surface of the non-woven graft material and at least one indentation in the top surface of the non-woven graft material. In another embodiment, the non-woven graft material can include at least one projection arising from a bottom surface of the non-woven graft material and at least one indentation in the bottom surface of the non-woven graft material. In another embodiment, the non-woven graft material can include at least one projection arising from a top surface of the non-woven graft material, at least one projection arising from a bottom surface of the non-woven graft material, at least one indentation in the top surface of the non-woven graft material, and at least one indentation in the bottom surface of the non-woven graft material. The plurality of indentations and the plurality of indentations can be patterned or randomly distributed on a surface of the non-woven graft material. Suitable methods for forming projections and indentations include pressing, stamping, and other methods known to those skilled in the art.

### Electrospinning

**In** some embodiments, the present disclosure is directed to methods of preparing the non-woven graft materials. The methods generally include preparing aqueous solutions of the polymers described above. Particularly, fibers resulting from separate polymer solutions can be contacted together using one or more processes such as electrospinning, electrospraying, melt-blowing, spunbonding, to form the non-woven graft material; and drying the non-woven graft material.

The non-woven graft material is dried to remove solvents used to prepare the aqueous polymer solutions. Drying can be done using methods generally known in the art, including, without limitation, Yankee dryers, vacuum chambers, vacuum ovens, and through-air dryers. Preferably, a non-compressive drying method that tends to preserve the bulk or thickness of the non-woven graft material is employed. Suitable through-drying apparatus and through-drying fabrics are conventional and well-known. One skilled in the art can readily determine the optimum drying gas temperature and residence time for a particular through-drying operation.

**In** some embodiments, a first fiber composition resulting from a first aqueous polymer solution and a second fiber composition resulting from a second aqueous polymer solution are blended to form a non-woven graft material using the electrospinning process as described above. The electrospinning process generally involves applying a high voltage (e.g., about 1 kV to about 100 kV, including about 3 kV to about 80 kV, depending on the configuration of the electrospinning apparatus) to a polymer fiber solution to produce a polymer jet. As the jet travels in air, the jet is elongated under repulsive electrostatic force to produce nanofibers or hybrid-scale fibers from the polymer fiber solution. The high voltage is applied between the grounded surface (or oppositely charged surface) and a conducting capillary into which a polymer fiber solution is injected. The high voltage can also be applied to the solution or melt through a wire if the capillary is a nonconductor such as a glass pipette. Initially the solution at the open tip of the capillary is pulled into a conical shape (the so-called "Taylor cone") through the interplay of electrical force and surface tension. At a certain voltage range, a fine jet of polymer fiber solution forms at the tip of the Taylor cone and shoots toward the target. Forces from the electric field accelerate and stretch the jet. This stretching, together with evaporation of solvent molecules, causes the jet diameter to become smaller. As the jet diameter decreases, the charge density increases until electrostatic forces within the polymer overcome the cohesive forces holding the jet together (e.g., surface tension), causing the jet to split or "splay" into a multifilament of polymer nanofibers or hybrid-scale fibers. The fibers continue to splay until they reach the collector, where they are collected as nonwoven nanofibers or hybrid-scale fibers, and are optionally dried.

Suitable solvents for preparing aqueous polymer solutions include, for example, hexafluoroisopropanol (HFIP), dichloromethane (DCM), dimethylformamide (DMF), acetone, and ethanol.

In some embodiments, the method can further include forming at least one projection arising from a surface of the non-woven graft material, forming at least one indentation in a surface of the non-woven graft material, and combinations thereof. The projection is a protrusion or bulge arising from a surface of the non-woven graft material. The projection can arise from a top surface of the non-woven graft material, a bottom surface of the non-woven graft material, and a top surface and a bottom surface of the non-woven graft material. The projection can be any desired shape such as, for example, circular, spherical, square, rectangular, diamond, star, irregular, and combinations thereof. The projection can be any desired height as measured from the surface of the material to the top of the projection. In one embodiment, the projection can have a substantially uniform height from the surface of the material. In another embodiment, the projection can further form gradually from the surface of the material to the highest measurable surface of the projection. In some embodiments, a surface of the non-woven graft material includes a plurality of protrusions. The plurality of protrusions can be patterned or randomly distributed on a surface of the non-woven graft material. In another embodiment, the method includes forming at least one indentation in a surface of the non-woven graft material. The indentation is a recess or depression in a surface of the non-woven graft material. The indentation can in a top surface of the non-woven graft material, a bottom surface of the non-woven graft material, and a top surface and a bottom surface of the non-woven graft material. The indentation can be any desired shape such as, for example, circular, spherical, square, rectangular, diamond, star, irregular, and combinations thereof. The indentation can be any desired depth as measured from the surface of the material to the bottom of the indentation. In one embodiment, the indentation can have a substantially uniform depth from the surface of the material to the deepest depth of the indentation. In another embodiment, the indentation can further form gradually from the surface of the material to the deepest depth of the indentation. In some embodiments, a surface of the non-woven graft material includes a plurality of indentations. The plurality of indentations can be patterned or randomly distributed on a surface of the non-woven graft material. In another embodiment, the non-woven graft material can include at least one projection arising from a surface of the non-woven graft material and at least one indentation in the surface of the non-woven graft material. In another embodiment, the non-woven graft material can include at least one projection arising from a top surface of the non-woven graft material and at least one indentation in the top surface of the non-woven graft material. In another embodiment, the non-woven graft material can include at least one projection arising from a bottom surface of the non-woven graft material and at least one indentation in the bottom surface of the non-woven graft material. In another embodiment, the non-woven graft material can include at least one projection arising from a top surface of the non-woven graft material, at least one projection arising from a bottom surface of the non-woven graft material, at least one indentation in the top surface of the non-woven graft material, and at least one indentation in the bottom surface of the non-woven graft material. The plurality of indentations and the plurality of indentations can be patterned or randomly distributed on a surface of the non-woven graft material. Suitable methods for forming projections and indentations include pressing, stamping, and other methods known to those skilled in the art.

In some embodiments, once the material has been electrospun, the macro perforations discussed above can be incorporated into the sheets of material, such as through a cutting step. This can be done mechanically, electronically, and/or computer controlled to provide for consistency in some embodiments. Laser cutting could also be used to create the perforations in the material.

In some embodiments, the perforations may be incorporated into the material during the electrospinning process, and thus no additional cutting step may be used. For example, a substrate pattern can be prepared so that when the fibers are electrospun onto the substrate, they leave a particular perforation pattern in the material. For example, the substrate can be metallic, and the fibers can stick to the metallic substrate to form the desired cut pattern.

In some embodiments, the sheets can be manufactured to have areas of higher or lower density of fibers, which can provide the macroporous abilities discussed herein.

### Tissue Repair

The present disclosure is directed to the skin graft for use in a method of tissue repair in an individual in need thereof. The method can include: applying a non-woven graft material to a surgical field, wherein the non-woven graft material comprises a first fiber composition and a second fiber composition. The method is particularly suitable for repairing tissues such as, for example, dura mater, pericardium, small intestinal submucosa, dermis, epidermis, tendon, trachea, heart valve leaflet, gastrointestinal tract, and cardiac tissue. Suitable tissue repair procedures include, for example, neurosurgeries such as dura mater repair, skin grafts, tracheal repair, gastrointestinal tract repair (e.g., abdominal hernia repair, ulcer repair), cardiac defect repair, head and neck surgeries, application to bone fractures, and burn repair.

Suitably, the non-woven graft material includes a first fiber composition, wherein the first fiber composition includes a polymer selected from polycaprolactone (poly(ε-caprolactone), PCL), polydioxanone (PDO), poly (glycolic acid) (PGA), poly(L-lactic acid) (PLA), poly(lactide-co-glycolide) (PLGA), poly(L-lactide) (PLLA), poly(D,L-lactide) (P(DLLA)), poly(ethylene glycol) (PEG), montmorillonite (MMT), poly(L-lactide-co-ε-caprolactone) (P(LLA-CL)), poly(ε-caprolactone-co-ethyl ethylene phosphate) (P(CL-EEP)), poly[bis(p-methylphenoxy) phosphazene] (PNmPh), poly(3-hydroxybutyrate-co-3-hydroxyvalerate) (PHBV), poly(ester urethane) urea (PEUU), poly(p-dioxanone) (PPDO), polyurethane (PU), polyethylene terephthalate (PET), poly(ethylene-co-vinylacetate) (PEVA), poly(ethylene oxide) (PEO), poly(phosphazene), poly(ethylene-co-vinyl alcohol), polymer nanoclay nanocomposites, poly(ethylenimine), poly(ethyleneoxide), poly vinylpyrrolidone; polystyrene (PS) and combinations thereof. Particularly suitable polymers include poly(lactic-co-glycolic acid), polydioxanone, polycaprolactone, and combinations thereof.

Suitably, the non-woven graft material includes a second fiber composition, wherein the second fiber composition includes a polymer selected from polycaprolactone (poly(ε-caprolactone), PCL), polydioxanone (PDO), poly (glycolic acid) (PGA), poly(L-lactic acid) (PLA), poly(lactide-co-glycolide) (PLGA), poly(L-lactide) (PLLA), poly(D,L-lactide) (P(DLLA)), poly(ethylene glycol) (PEG), montmorillonite (MMT), poly(L-lactide-co-ε-caprolactone) (P(LLA-CL)), poly(ε-caprolactone-co-ethyl ethylene phosphate) (P(CL-EEP)), poly[bis(p-methylphenoxy) phosphazene] (PNmPh), poly(3-hydroxybutyrate-co-3-hydroxyvalerate) (PHBV), poly(ester urethane) urea (PEUU), poly(p-dioxanone) (PPDO), polyurethane (PU), polyethylene terephthalate (PET), poly(ethylene-co-vinylacetate) (PEVA), poly(ethylene oxide) (PEO), poly(phosphazene), poly(ethylene-co-vinyl alcohol), polymer nanoclay nanocomposites, poly(ethylenimine), poly(ethyleneoxide), poly vinylpyrrolidone; polystyrene (PS) and combinations thereof. Particularly suitable polymers include poly(lactic-co-glycolic acid), polydioxanone, polycaprolactone, and combinations thereof.

**In** a particularly suitable embodiment, the non-woven graft material includes a first fiber composition comprising poly(lactic-co-glycolic acid) and a second fiber composition comprising polydioxanone.

As used herein, "individual in need thereof" refers to an individual having a tissue defect, tissue damage, tissue that is missing due to damage or removal, and tissue damaged by incision. The methods are particularly suitable for use with an individual or subset of individuals having dura defects requiring repair of the dura mater. Individuals having dura defects can be those having a perforation in the dura mater, those having dura mater removed, those having damaged dura mater, and those having dura mater with a surgical incision. The individual in need thereof can be an adult individual, a child, and a pediatric individual. Particularly suitable individuals can be a human. Other particularly suitable individuals can be animals such as primates, pigs, dogs, cats, rabbits, rodents (e.g., mice and rats), and the like.

Moreover, while methods may be depicted in the drawings or described in the specification in a particular order, such methods need not be performed in the particular order shown or in sequential order, and that all methods need not be performed, to achieve desirable results. Other methods that are not depicted or described can be incorporated in the example methods and processes. For example, one or more additional methods can be performed before, after, simultaneously, or between any of the described methods. Further, the methods may be rearranged or reordered in other implementations. Also, the separation of various system components in the implementations described above should not be understood as requiring such separation in all implementations, and it should be understood that the described components and systems can generally be integrated together in a single product or packaged into multiple products. Additionally, other implementations are within the scope of this disclosure.

Conditional language, such as "can," "could," "might," or "may," unless specifically stated otherwise, or otherwise understood within the context as used, is generally intended to convey that certain embodiments include or do not include, certain features, elements, and/or steps. Thus, such conditional language is not generally intended to imply that features, elements, and/or steps are in any way required for one or more embodiments.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than or equal to 10% of, within less than or equal to 5% of, within less than or equal to 1% of, within less than or equal to 0.1% of, and within less than or equal to 0.01% of the stated amount. If the stated amount is 0 (e.g., none, having no), the above recited ranges can be specific ranges, and not within a particular % of the value. For example, within less than or equal to 10 wt./vol. % of, within less than or equal to 5 wt./vol. % of, within less than or equal to 1 wt./vol. % of, within less than or equal to 0.1 wt./vol. % of, and within less than or equal to 0.01 wt./vol. % of the stated amount. Additionally, all values of tables within the disclosure are understood to either be the stated values or, alternatively, about the stated value.

## Claims

1. A three-dimensional electrospun hybrid-scale fiber matrix synthetic skin graft for use in repairing tissue for wound care, the three-dimensional hybrid-scale fiber matrix synthetic skin graft comprising:
a flexible electrospun fiber network, the flexible electrospun fiber network comprising:
a first set of electrospun fibers comprising a first bioresorbable polymer; and
a second set of electrospun fibers comprising a second bioresorbable polymer,
wherein the first bioresorbable polymer comprises a different composition from the second bioresorbable polymer;
the flexible electrospun fiber network further comprising one or more macro-scale pores and one or more micro-scale pores, the one or more macro-scale pores comprising an opening of 1 mm ± 10 % to 20 mm ± 10 %, and the one or more micro-scale pores comprising an opening with areas of 10 µm² ± 10 % to less than 300 µm²,
wherein the three-dimensional electrospun hybrid-scale fiber matrix synthetic skin graft is sufficiently flexible to facilitate application of the three-dimensional electrospun hybrid-scale fiber matrix synthetic skin graft to uneven surfaces of the tissue,
wherein the three-dimensional electrospun hybrid-scale fiber matrix synthetic skin graft is sufficiently flexible to enable movement of the three-dimensional electrospun hybrid-scale fiber matrix synthetic skin graft by the tissue,
wherein the first set of electrospun fibers comprise an average diameter less than 10 micrometers, optionally wherein the first set of electrospun fibers comprise an average diameter not greater than 2,000 nanometers, and
wherein the first set of electrospun fibers and the second set of electrospun fibers are configured to degrade after application to the tissue.

2. The three-dimensional electrospun hybrid-scale fiber matrix synthetic skin graft of Claim 1, wherein the one or more macro-scale pores of the three-dimensional electrospun hybrid-scale fiber matrix synthetic skin graft are configured to allow flow through of an exudate.

3. The three-dimensional electrospun hybrid-scale fiber matrix synthetic skin graft of Claim 1, wherein the one or more micro-scale pores of the three-dimensional electrospun hybrid-scale fiber matrix synthetic skin graft are configured to facilitate cell growth.

4. The three-dimensional electrospun hybrid-scale fiber matrix synthetic skin graft of Claim 1, wherein the first bioresorbable polymer comprises poly(lactic-co-glycolic acid), and wherein the second bioresorbable polymer comprises polydioxanone.

5. The three-dimensional electrospun hybrid-scale fiber matrix synthetic skin graft of Claim 1, wherein perforations are distributed equally throughout the matrix.

6. The three-dimensional electrospun hybrid-scale fiber matrix synthetic skin graft of Claim 1, wherein the three-dimensional electrospun hybrid-scale fiber matrix synthetic skin graft comprises a plurality of protrusions arising from a surface of the flexible electrospun fiber network and a plurality of indentations in a surface of the flexible electrospun fiber network, wherein the plurality of protrusions arising from the surface comprise a substantially uniform height, wherein the plurality of indentations in thesurface comprise a substantially uniform depth, wherein the plurality of protrusions and the plurality of indentations are spherical, wherein the plurality of protrusions are patterned throughout the surface, and wherein the plurality of indentations are patterned throughout the surface.

7. The three-dimensional electrospun hybrid-scale fiber matrix synthetic skin graft of Claim 1, wherein the one or more macro-scale pores comprise slits through a full thickness of the three-dimensional hybrid-scale fiber matrix synthetic skin graft with a density of at least 0.78 slits per cm² (5 slits per square inch).

8. A method of manufacturing a biomedical patch device for tissue repair, the method comprising:
depositing a first structure of fibers having electrospun hybrid-scale fibers via electrospinning, the first structure of fibers configured to promote cell growth; and
depositing a second structure of fibers having electrospun hybrid-scale fibers via electrospinning, the second structure of fibers configured to promote cell growth,
the first structure of fibers comprising a different composition from the second structure of fibers;
the first structure of fibers and the second structure of fibers comprising one or more macro-scale pores and one or more micro-scale pores, the one or more macro-scale pores comprising an opening of 1 mm ± 10 % to 20 mm ± 10 %, and the one or more micro-scale pores comprising an opening with areas of 10 µm² ± 10 % to less than 300 µm²;
the biomedical patch device comprising a surface, wherein the surface comprises a surface pattern configured to contact tissue, wherein the surface pattern, the first structure of fibers, and the second structure of fibers are configured to promote cell growth in one or more defined directions, the biomedical patch device sufficiently flexible to facilitate application of the biomedical patch device to uneven surfaces of the tissue, the biomedical patch device sufficiently flexible to enable movement of the biomedical patch device with the tissue,
wherein the first structure of fibers comprise an average diameter less than 10 micrometers, optionally wherein the first structure of fibers comprise an average diameter not greater than 2,000 nanometers, and
wherein the first structure of fibers and the second structure of fibers are configured to degrade after application to the tissue.

9. The method of Claim 8, wherein a first portion of the biomedical patch of a particular size comprises a higher number of fibers than a second portion of the biomedical patch of the particular size.

10. The method of Claim 8, wherein the surface pattern is formed by positioning a mask between a collector and a spinneret, wherein the mask is configured to prevent depositing at least some of the first structure of fibers or the second structure of fibers on the collector.

11. The method of Claim 8, wherein the surface pattern is formed by depositing the first structure of fibers and the second structure of fibers directly on a collector without a mask.

12. The method of Claim 11, wherein the surface pattern comprises a plurality of organized features or comprises a plurality of topographical features configured to further promote migration of cells in one or more of the plurality of defined directions.

13. The method of Claim 8, wherein the macro-scale pores are generated through cutting mechanically, electronically, and/or computer controlled, optionally wherein the cutting is laser cutting.

14. The method of Claim 8, wherein the surface comprises a top surface and a bottom surface, and wherein the biomedical patch device comprises a plurality of protrusions arising from a surface and a plurality of indentations in a surface, wherein the plurality of protrusions arising from the surface comprise a substantially uniform height, wherein the plurality of indentations in the surface comprise a substantially uniform depth, wherein the plurality of protrusions and the plurality of indentations are spherical, wherein the plurality of protrusions are patterned throughout the surface, and wherein the plurality of indentations are patterned throughout the surface.

15. The method of Claim 8, further comprising forming the one or more macro-scale pores as slits through a full thickness of the three-dimensional hybrid-scale fiber matrix synthetic skin graft with a density of at least 0.78 slits per cm² (5 slits per square inch).

## Patentansprüche

1. Ein dreidimensionales, elektrogesponnenes, synthetisches Hauttransplantat mit synthetischer Fasermatrix im Hybridmaßstab zur Verwendung bei einem Reparieren von Gewebe für die Wundversorgung, das dreidimensionale, elektrogesponnene, synthetische Hauttransplantat mit synthetischer Fasermatrix im Hybridmaßstab aufweisend:
ein flexibles elektrogesponnenes Fasernetzwerk, das flexible elektrogesponnene Fasernetzwerk aufweisend:
einen ersten Satz elektrogesponnener Fasern, aufweisend ein erstes bioresorbierbares Polymer; und
einen zweiten Satz elektrogesponnener Fasern, aufweisend ein zweites bioresorbierbares Polymer,
wobei das erste bioresorbierbare Polymer eine unterschiedliche Zusammensetzung als das zweite bioresorbierbare Polymer aufweist;
wobei das flexible elektrogesponnene Fasernetzwerk ferner eine oder mehrere Poren im Makromaßstab und eine oder mehrere Poren im Mikromaßstab aufweisen, wobei die eine oder mehreren Poren im Makromaßstab eine Öffnung von 1 mm ± 10 % bis 20 mm ± 10 % aufweisen,
und die eine oder mehreren Poren im Mikromaßstab Öffnung mit Flächen von 10 µm² ± 10 % bis weniger als 300 µm² aufweisen,
wobei das dreidimensionale, elektrogesponnene, synthetische Hauttransplantat mit synthetischer Fasermatrix im Hybridmaßstab ausreichend flexibel ist, um die Aufbringung des dreidimensionalen elektrogesponnenen synthetischen Hauttransplantats mit synthetischer Fasermatrix im Hybridmaßstab auf unebene Oberflächen des Gewebes zu vereinfachen,
wobei das dreidimensionale, elektrogesponnene, synthetische Hauttransplantat mit synthetischer Fasermatrix im Hybridmaßstab ausreichend flexibel ist, um die Bewegung des dreidimensionalen elektrogesponnenen synthetischen Hauttransplantats mit synthetischer Fasermatrix im Hybridmaßstab durch das Gewebe zu ermöglichen,
wobei der erste Satz elektrogesponnener Fasern einen durchschnittlichen Durchmesser von weniger als 10 Mikrometer aufweist, optional wobei der erste Satz elektrogesponnener Fasern einen durchschnittlichen Durchmesser von nicht mehr als 2.000 Nanometer aufweist, und
wobei der erste Satz elektrogesponnener Fasern und der zweite Satz elektrogesponnener Fasern konfiguriert sind, um sich nach der Aufbringung auf das Gewebe abzubauen.

2. Das dreidimensionale, elektrogesponnene, synthetische Hauttransplantat mit synthetischer Fasermatrix im Hybridmaßstab nach Anspruch 1, wobei die eine oder mehreren Poren im Makromaßstab des dreidimensionalen elektrogesponnenen synthetischen Hauttransplantats mit synthetischer Fasermatrix im Hybridmaßstab konfiguriert sind, um den Durchfluss eines Exsudats zu erlauben.

3. Das dreidimensionale, elektrogesponnene, synthetische Hauttransplantat mit synthetischer Fasermatrix im Hybridmaßstab nach Anspruch 1, wobei die eine oder mehreren Poren im Makromaßstab des dreidimensionalen elektrogesponnenen synthetischen Hauttransplantats mit synthetischer Fasermatrix im Hybridmaßstab konfiguriert sind, um Zellwachstum zu vereinfachen.

4. Das dreidimensionale, elektrogesponnene, synthetische Hauttransplantat mit synthetischer Fasermatrix im Hybridmaßstab nach Anspruch 1, wobei das erste bioresorbierbare Polymer Poly(lactid-co-glykolsäure) aufweist, und wobei das zweite bioresorbierbare Polymer Polydioxanon aufweist.

5. Das dreidimensionale, elektrogesponnene, synthetische Hauttransplantat mit synthetischer Fasermatrix im Hybridmaßstab nach Anspruch 1, wobei Perforationen in der Matrix durchweg gleichmäßig verteilt sind.

6. Das dreidimensionale, elektrogesponnene, synthetische Hauttransplantat mit synthetischer Fasermatrix im Hybridmaßstab nach Anspruch 1, wobei das dreidimensionale, elektrogesponnene, synthetische Hauttransplantat mit synthetischer Fasermatrix im Hybridmaßstab eine Mehrzahl von Vorsprüngen, die sich von einer Oberfläche des flexiblen elektrogesponnenen Fasernetzwerks erheben, und eine Mehrzahl von Vertiefungen in einer Oberfläche des flexiblen elektrogesponnenen Fasernetzwerks aufweist, wobei die Mehrzahl von Vorsprüngen, die sich von der Oberfläche erheben, eine im Wesentlichen einheitliche Höhe aufweisen, wobei die Mehrzahl von Vertiefungen in der Oberfläche eine im Wesentlichen einheitliche Tiefe aufweisen, wobei die Mehrzahl von Vorsprüngen und die Mehrzahl von Vertiefungen kugelförmig sind, wobei die Mehrzahl von Vorsprüngen über die Oberfläche durchweg gemustert sind, und wobei die Mehrzahl von Vertiefungen über die Oberfläche durchweg gemustert sind.

7. Das dreidimensionale, elektrogesponnene, synthetische Hauttransplantat mit synthetischer Fasermatrix im Hybridmaßstab nach Anspruch 1, wobei die eine oder mehreren Poren im Makromaßstab Schlitze durch eine volle Dicke des dreidimensionalen, synthetischen Hauttransplantat mit synthetischer Fasermatrix im Hybridmaßstab mit einer Dichte von mindestens 0,78 Schlitzen pro cm² (5 Schlitze pro Quadratzoll) aufweisen.

8. Ein Verfahren zum Herstellen einer biomedizinischen Pflastervorrichtung für eine Gewebereparatur, das Verfahren aufweisend:
Ablagern einer ersten Struktur von Fasern, aufweisend elektrogesponnene Fasern im Hybridmaßstab, über Elektrospinnen, wobei die erste Struktur von Fasern konfiguriert ist, um Zellwachstum zu fördern; und Ablagern einer zweiten Struktur von Fasern, aufweisend elektrogesponnene Fasern im Hybridmaßstab, über Elektrospinnen, wobei die zweite Struktur von Fasern konfiguriert ist, um Zellwachstum zu fördern, die erste Struktur aufweisend Fasern eine unterschiedlichen Zusammensetzung als die zweite Struktur von Fasern;
wobei die erste Struktur von Fasern und die zweite Struktur von Fasern eine oder mehrere Poren im Makromaßstab und eine oder mehrere Poren im Mikromaßstab aufweisen, wobei die eine oder mehreren Poren im Makromaßstab eine Öffnung von 1 mm ± 10 % bis 20 mm ± 10 % aufweisen,
und die eine oder mehreren Poren im Mikromaßstab eine Öffnung mit Flächen von 10 µm² ± 10 % bis weniger als 300 µm² aufweisen;
wobei die biomedizinische Pflastervorrichtung eine Oberfläche aufweist, wobei die Oberfläche ein Oberflächenmuster aufweist, das konfiguriert ist, um Gewebe zu kontaktieren, wobei das Oberflächenmuster, die erste Struktur von Fasern und die zweite Struktur von Fasern konfiguriert sind, um Zellwachstum in einer oder mehreren definierten Richtungen zu fördern, wobei die biomedizinische Pflastervorrichtung ausreichend flexibel ist, um die Aufbringung der biomedizinischen Pflastervorrichtung auf unebene Oberflächen des Gewebes zu vereinfachen, wobei die biomedizinische Pflastervorrichtung ausreichend flexibel ist, um eine Bewegung der biomedizinischen Pflastervorrichtung mit dem Gewebe zu ermöglichen,
wobei die erste Struktur von Fasern einen durchschnittlichen Durchmesser von weniger als 10 Mikrometer aufweist, optional wobei die erste Struktur von Fasern einen durchschnittlichen Durchmesser von nicht mehr als 2.000 Nanometer aufweist, und
wobei die erste Struktur von Fasern und die zweite Struktur von Fasern konfiguriert sind, um sich nach der Aufbringung auf das Gewebe abzubauen.

9. Das Verfahren nach Anspruch 8, wobei ein erster Abschnitt des biomedizinischen Pflasters einer bestimmten Größe eine höhere Anzahl von Fasern aufweist als ein zweiter Abschnitt des biomedizinischen Pflasters der bestimmten Größe.

10. Das Verfahren nach Anspruch 8, wobei das Oberflächenmuster durch Positionieren einer Maske zwischen einem Kollektor und einer Spinndüse ausgebildet wird, wobei die Maske konfiguriert ist, um das Ablagern von mindestens einem Teil der ersten Struktur von Fasern oder der zweiten Struktur von Fasern auf dem Kollektor zu verhindern.

11. Das Verfahren nach Anspruch 8, wobei das Oberflächenmuster durch Ablagern der ersten Struktur von Fasern und der zweiten Struktur von Fasern direkt auf einem Kollektor ohne eine Maske ausgebildet wird.

12. Das Verfahren nach Anspruch 11, wobei das Oberflächenmuster eine Mehrzahl von organisierten Merkmalen aufweist oder eine Mehrzahl von topografischen Merkmalen aufweist, die konfiguriert sind, um die Migration von Zellen in eine oder mehrere der Mehrzahl von definierten Richtungen weiter zu fördern.

13. Das Verfahren nach Anspruch 8, wobei die Poren im Makromaßstab durch mechanisches, elektronisches und/oder computergesteuertes Schneiden erzeugt werden, optional wobei das Schneiden Laserschneiden ist.

14. Das Verfahren nach Anspruch 8, wobei die Oberfläche eine obere Oberfläche und eine untere Oberfläche aufweist, und wobei die biomedizinische Pflastervorrichtung eine Mehrzahl von Vorsprüngen, die sich von einer Oberfläche erheben, und eine Mehrzahl von Vertiefungen in einer Oberfläche aufweist, wobei die Mehrzahl von Vorsprüngen, die sich von der Oberfläche erheben, eine im Wesentlichen einheitliche Höhe aufweisen, wobei die Mehrzahl von Vertiefungen in der Oberfläche eine im Wesentlichen einheitliche Tiefe aufweisen, wobei die Mehrzahl von Vorsprüngen und die Mehrzahl von Vertiefungen kugelförmig sind, wobei die Mehrzahl von Vorsprüngen über die Oberfläche durchweg gemustert sind, und wobei die Mehrzahl von Vertiefungen über die Oberfläche durchweg gemustert sind.

15. Das Verfahren nach Anspruch 8, ferner aufweisend das Ausbilden der einen oder mehreren Poren im Makromaßstab als Schlitze durch eine gesamte Dicke des dreidimensionalen, synthetischen Hauttransplantats mit synthetischer Fasermatrix mit einer Dichte von mindestens 0,78 Schlitzen pro cm² (5 Schlitze pro Quadratzoll).

## Revendications

1. Greffon de peau synthétique à matrice de fibres d'échelle hybride électrofilées tridimensionnelle destiné à être utilisé dans la réparation de tissus pour le soin de plaie, le greffon de peau synthétique à matrice de fibres d'échelle hybride tridimensionnelle comprenant :
un réseau de fibres électrofilées flexible, le réseau de fibres électrofilées flexible comprenant :
un premier ensemble de fibres électrofilées comprenant un premier polymère biorésorbable ; et
un second ensemble de fibres électrofilées comprenant un second polymère biorésorbable,
dans lequel le premier polymère biorésorbable comprend une composition différente du second polymère biorésorbable ;
le réseau de fibres électrofilées flexible comprenant en outre un ou plusieurs pores d'échelle macroscopique et un ou plusieurs pores d'échelle microscopique, le ou les pores d'échelle macroscopique comprenant une ouverture de 1 mm ± 10 % à 20 mm ± 10 %,
et le ou les pores d'échelle microscopique comprenant une ouverture avec des aires de 10 µm² + 10 % à moins de 300 µm²,
dans lequel le greffon de peau synthétique à matrice de fibres d'échelle hybride électrofilées tridimensionnelle est suffisamment flexible pour faciliter l'application du greffon de peau synthétique à matrice de fibres d'échelle hybride électrofilées tridimensionnelle sur des surfaces irrégulières du tissu,
dans lequel le greffon de peau synthétique à matrice de fibres d'échelle hybride électrofilées tridimensionnelle est suffisamment flexible pour permettre le déplacement du greffon de peau synthétique à matrice de fibres d'échelle hybride électrofilées tridimensionnelle par le tissu,
dans lequel le premier ensemble de fibres électrofilées comprend un diamètre moyen inférieur à 10 micromètres, éventuellement dans lequel le premier ensemble de fibres électrofilées comprend un diamètre moyen non supérieur à 2 000 nanomètres, et
dans lequel le premier ensemble de fibres électrofilées et le second ensemble de fibres électrofilées sont conçus pour se dégrader après application sur le tissu.

2. Greffon de peau synthétique à matrice de fibres d'échelle hybride électrofilées tridimensionnelle selon la revendication 1, dans lequel le ou les pores d'échelle macroscopique du greffon de peau synthétique à matrice de fibres d'échelle hybride électrofilées tridimensionnelle sont conçus pour permettre l'écoulement traversant d'un exsudat.

3. Greffon de peau synthétique à matrice de fibres d'échelle hybride électrofilées tridimensionnelle selon la revendication 1, dans lequel le ou les pores d'échelle microscopique du greffon de peau synthétique à matrice de fibres d'échelle hybride électrofilées tridimensionnelle sont conçus pour faciliter la croissance cellulaire.

4. Greffon de peau synthétique à matrice de fibres d'échelle hybride électrofilées tridimensionnelle selon la revendication 1, dans lequel le premier polymère biorésorbable comprend du poly(acide lactique-co-glycolique), et dans lequel le second polymère biorésorbable comprend de la polydioxanone.

5. Greffon de peau synthétique à matrice de fibres d'échelle hybride électrofilées tridimensionnelle selon la revendication 1, dans lequel les perforations sont réparties de manière égale dans toute la matrice.

6. Greffon de peau synthétique à matrice de fibres d'échelle hybride électrofilées tridimensionnelle selon la revendication 1, dans lequel le greffon de peau synthétique à matrice de fibre d'échelle hybride électrofilées tridimensionnelle comprend une pluralité de protubérances émergeant d'une surface du réseau de fibres électrofilées flexibles et une pluralité d'indentations dans une surface du réseau de fibres électrofilées flexibles, dans lequel la pluralité de protubérances émergeant de la surface comprennent une hauteur sensiblement uniforme, dans lequel la pluralité d'indentations dans la surface comprennent une profondeur sensiblement uniforme, dans lequel la pluralité de protubérances et la pluralité d'indentations sont sphériques, dans lequel la pluralité de protubérances sont structurées sur toute la surface, et dans lequel la pluralité d'indentations sont structurées sur toute la surface.

7. Greffon de peau synthétique à matrice de fibres d'échelle hybride électrofilées tridimensionnelle selon la revendication 1, dans lequel le ou les pores d'échelle macroscopique comprennent des fentes sur toute l'épaisseur du greffon de peau synthétique à matrice de fibres d'échelle hybride tridimensionnelle avec une densité d'au moins 0,78 fente par cm² (5 fentes par pouce carré).

8. Procédé de fabrication d'un dispositif de timbre biomédical pour la réparation de tissu, le procédé comprenant :
le dépôt d'une première structure de fibres ayant des fibres d'échelle hybride électrofilées par le biais d'électrofilage, la première structure de fibres étant conçue pour promouvoir la croissance cellulaire ; et le dépôt d'une seconde structure de fibres ayant des fibres d'échelle hybride électrofilées par le biais d'électrofilage, la seconde structure de fibres étant conçue pour promouvoir la croissance cellulaire, la première structure de fibres comprenant une composition différente de la seconde structure de fibres ;
la première structure de fibres et la seconde structure de fibres comprenant un ou plusieurs pores d'échelle macroscopique et un ou plusieurs pores d'échelle microscopique, le ou les pores d'échelle macroscopique comprenant une ouverture de 1 mm ± 10 % à 20 mm ± 10 %,
et le ou les pores d'échelle microscopique comprenant une ouverture avec des aires de 10 µm² + 10 % à moins de 300 µm² ;
le dispositif de timbre biomédical comprenant une surface, dans lequel la surface comprend un motif de surface conçu pour entrer en contact avec le tissu, dans lequel le motif de surface, la première structure de fibres et la seconde structure de fibres sont conçus pour promouvoir la croissance cellulaire dans une ou plusieurs directions définies, le dispositif de timbre biomédical étant suffisamment flexible pour faciliter l'application du dispositif de timbre biomédical sur des surfaces inégales du tissu, le dispositif de timbre biomédical étant suffisamment flexible pour permettre un déplacement du dispositif de timbre biomédical avec le tissu,
dans lequel la première structure de fibres comprend un diamètre moyen inférieur à 10 micromètres, éventuellement dans lequel la première structure de fibres comprend un diamètre moyen non supérieur à 2 000 nanomètres, et
dans lequel la première structure de fibres et la seconde structure de fibres sont conçues pour se dégrader après application sur le tissu.

9. Procédé selon la revendication 8, dans lequel une première partie du timbre biomédical d'une taille particulière comprend un nombre de fibres plus élevé qu'une seconde partie du timbre biomédical de la taille particulière.

10. Procédé selon la revendication 8, dans lequel le motif de surface est formé par positionnement d'un masque entre un collecteur et une buse à filer, dans lequel le masque est conçu pour empêcher le dépôt d'au moins une partie de la première structure de fibres ou de la seconde structure de fibres sur le collecteur.

11. Procédé selon la revendication 8, dans lequel le motif de surface est formé par dépôt de la première structure de fibres et de la seconde structure de fibres directement sur un collecteur sans masque.

12. Procédé selon la revendication 11, dans lequel le motif de surface comprend une pluralité de caractéristiques organisées ou comprend une pluralité de caractéristiques topographiques conçues pour promouvoir davantage la migration des cellules dans une ou plusieurs de la pluralité de directions définies.

13. Procédé selon la revendication 8, dans lequel les pores d'échelle macroscopique sont générés par découpe mécanique, électronique et/ou commandée par ordinateur, éventuellement dans lequel la découpe est une découpe au laser.

14. Procédé selon la revendication 8, dans lequel la surface comprend une surface haute et une surface basse, et dans lequel le dispositif biomédical de timbre comprend une pluralité de protubérances émergeant d'une surface et une pluralité d'indentations dans une surface, dans lequel la pluralité de protubérances émergeant de la surface comprennent une hauteur sensiblement uniforme, dans lequel la pluralité d'indentations dans la surface comprennent une profondeur sensiblement uniforme, dans lequel la pluralité de protubérances et la pluralité d'indentations sont sphériques, dans lequel la pluralité de protubérances sont structurées sur toute la surface, et dans lequel la pluralité d'indentations sont structurées sur toute la surface.

15. Procédé selon la revendication 8, comprenant en outre la formation du ou des pores d'échelle macroscopique sous forme de fentes à travers une épaisseur complète du greffon de peau synthétique à matrice de fibres d'échelle hybride tridimensionnelle avec une densité d'au moins 0,78 fente par cm² (5 fentes par pouce carré).
